# EUROPÄISCHE PATENTSCHRIFT

(11) **EP 0 672 396 B1**
(45) Veröffentlichungstag und Bekanntmachung des Hinweises auf die Patenterteilung: **19.08.1998**
(21) Anmeldenummer: 95101573.4
(22) Anmeldetag: 07.02.1995
(51) Int. Cl.: A61F 2/36, A61F 2/46

(54) **Femorale Schaftendoprothese**
Femoral shaft prosthesis
Tige fémorale

(30) Priorität: 26.02.1994 DE 4406388
(43) Veröffentlichungstag der Anmeldung: 20.09.1995
(73) Patentinhaber: AESCULAP AG, 78532 Tuttlingen (DE)
(72) Erfinder: Hermle, Thomas, D-786628 Rottweil (DE)
(74) Vertreter: Hoeger, Stellrecht & Partner

(56) Entgegenhaltungen:
- EP-A- 0 135 755
- EP-A- 0 499 480
- DE-U- 9 110 907
- GB-A- 1 126 961
- US-A- 4 546 501

## Beschreibung

Die Erfindung betrifft eine Schaftendoprothese für Röhrenknochen mit einem im Querschnitt kreisbogenförmigen Schaft, dessen Längsachse in einer ersten Ebene verläuft, mit einem an den oberen Teil des Schaftes einseitig angeformten, sich in einer zweiten, senkrecht zur ersten verlaufenden Ebene erstreckenden und sich nach oben hin erweiternden Ansatz, der auf der dem Schaft abgewandten Seite bogenförmig begrenzt wird und dessen Dicke quer zur zweiten Ebene dem Durchmesser des Schaftes entspricht, und mit einem sich an die Oberseite des Ansatzes anschließenden, in der zweiten Ebene liegenden, eine Gelenkkugel aufnehmenden, gegenüber der Längsachse des Schaftes geneigten Hals.

Eine solche Schaftendoprothese ist beispielsweise aus dem europäischen Patent 359 097 bekannt.

Ausgehend von dieser vorbekannten Schaftendoprothese liegt der Erfindung die Aufgabe zugrunde, eine derartige Schaftendoprothese so weiterzubilden, daß sie optimal an anatomische Verhältnisse angepaßt werden kann. Insbesondere soll eine möglichst großflächige Kraftübertragung erreicht werden, um Spannungsspitzen im Knochen zu vermeiden.

Diese Aufgabe wird bei einer Schaftendoprothese der eingangs beschriebenen Art erfindungsgemäß dadurch gelöst, daß der untere Teil des Schaftes gegenüber der zweiten Ebene um einen Winkel zwischen 5° und 15° geneigt ist und daß an den oberen Teil des Schaftes eine sich längs des Schaftes und senkrecht zur zweiten Ebene über die konkave Außenfläche erhebende Rippe angeformt ist.

Die Neigung des unteren Schaftteiles gegenüber der zweiten Ebene, in der sich der obere Teil des Schaftes und der Ansatz befinden, führt dazu, daß ein Anteversionswinkel zwischen 5° und 15° erhalten wird. Die auf die innenliegende, konkave Außenfläche aufgesetzte und längs des Schaftes verlaufende Rippe legt sich besonders günstig an die Innenseite des entsprechend vorbereiteten Markraumes eines Femurknochens an, so daß eine besonders große Kontaktfläche erzielt werden kann.

Günstig ist es dabei, wenn der Winkel zwischen dem unteren Teil des Schaftes und der zweiten Ebene bei etwa 10° liegt.

Der untere Teil des Schaftes ist geradlinig ausgebildet, der obere Teil des Schaftes, der in der zweiten Ebene liegt, ebenfalls. Im Übergangsbereich ist es günstig, wenn der Schaft in diesem Bereich kontinuierlich gebogen ist.

Auch der Teil des Ansatzes, der in dem Übergangsbereich angeordnet ist, kann in gleicher Weise wie der Schaft kontinuierlich gebogen sein.

Dabei ist vorgesehen, daß der Übergang von dem geradlinigen unteren Teil des Schaftes in die dagegen geneigte zweite Ebene etwa in der Mitte der Endoprothese erfolgt.

Günstig ist es, wenn sich die Rippe etwa bis zum Beginn des unteren, geradlinigen, gegenüber der zweiten Ebene geneigten Teil des Schaftes erstreckt.

Bei einer bevorzugten Ausführungsform ist vorgesehen, daß die Höhe der Rippe gegenüber der Außenfläche des Schaftes von oben nach unten abnimmt.

Am oberen Ende des Schaftes kann eine Verbindungsstelle für ein Einsetzwerkzeug angeordnet sein.

Die nachfolgende Beschreibung einer bevorzugten Ausführungsform der Erfindung dient im Zusammenhang mit der Zeichnung der näheren Erläuterung. Es zeigen:
- Figur 1:: eine Seitenansicht einer Schaftendoprothese ohne Gelenkkugel mit aufgesetztem Einsetzwerkzeug;
- Figur 2:: eine Vorderansicht der Schaftendoprothese der Figur 1 ohne umgebenden Knochen;

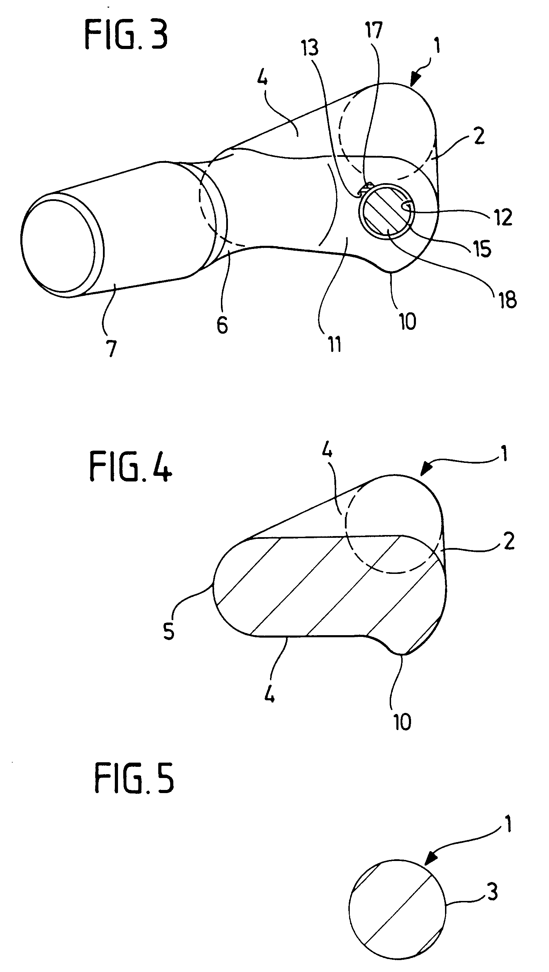

- Figur 3:: eine Schnittansicht längs Linie 3-3 in Figur 2;
- Figur 4:: eine Schnittansicht längs Linie 4-4 in Figur 2;
- Figur 5:: eine Schnittansicht längs Linie 5-5 in Figur 2;
- Figur 6:: eine Längsschnittansicht eines Einsetzwerkzeuges und
- Figur 7:: eine vergrößerte Teillängsschnittansicht des Verbindungsbereiches zwischen Einsetzwerkzeug und Schaftendoprothese.

Die in der Zeichnung dargestellte Endoprothese umfaßt einen Stiel oder Schaft 1 mit kreisförmigem Querschnitt. Dieser Schaft 1 erstreckt sich über die gesamte Höhe der Prothese und besteht aus zwei geradlinigen Abschnitten, nämlich einem oberen Teil 2 und einem unteren Teil 3. Diese beiden geradlinigen Teile des Schaftes 1 sind etwa in der Mitte des Schaftes gegeneinander in einem Winkel zwischen 5° und 15° geneigt.

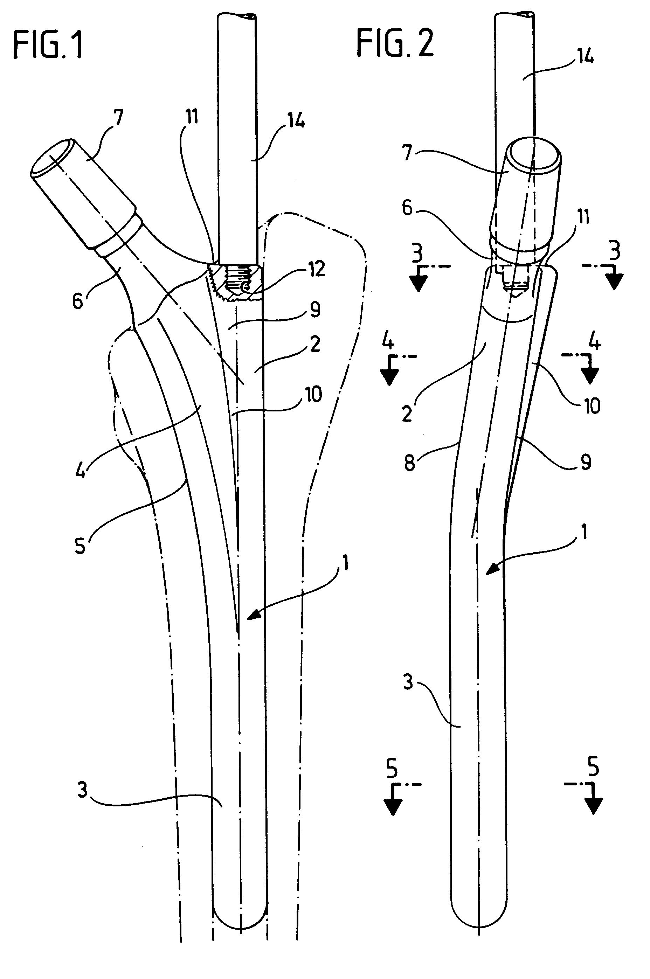

Die Längsachse des Schaftes 1 liegt somit in einer ersten Ebene, die senkrecht auf der Zeichnungsebene der Figur 1 liegt, die Längsachse des oberen Teiles des Schaftes 1 liegt in einer zweiten Ebene, die senkrecht auf dieser ersten Ebene steht. Gegenüber dieser zweiten Ebene ist der untere Teil 3 des Schaftes 1 um den genannten Winkel geneigt. Dabei gehen oberer Teil 2 und unterer Teil 3 des Schaftes 1 kontinuierlich ineinander über, d. h. im Übergangsbereich ist der Schaft kontinuierlich gebogen.

An den oberen Teil 2 ist an einer Seite ein Ansatz 4 angeformt, der sich in der zweiten Ebene erstreckt. Die Dicke des Ansatzes 4, also die Ausdehnung senkrecht zu dieser zweiten Ebene, entspricht dem Durchmesser des Schaftes 1, so daß der Schaft 1 mit seinem oberen Teil kontinuierlich in den Ansatz 4 übergeht. Auf der dem Schaft 1 abgewandten Seite wird der Ansatz 4 begrenzt durch eine bogenförmige Kontur 5, die in den unteren Teil des Schaftes 3 übergeht und die sich nach oben hin vom Schaft 1 entfernt, so daß sich der Ansatz 4 von unten nach oben hin erweitert. Im Randbereich ist der Ansatz 4 durch eine halbkreisförmige Kontur begrenzt, die vollständig der halbkreisförmigen Kontur des Schaftes 1 entspricht.

Der Ansatz 4 reicht bis in den obersten Bereich des unteren Teiles 3 des Schaftes 1 hinein und ist in gleicher Weise wie der Schaft 1 etwa in der Mitte der Prothese abgewinkelt.

An seinem oberen Ende trägt der Ansatz 4 einen Hals 6 mit einem Steckkonus 7 für einen in der Zeichnungen nicht dargestellten Gelenkkopf, sowohl der Hals 6 als auch der Steckkonus 7 liegen in der zweiten Ebene. Die Längsachse des Halses 6 schließt mit der Längsachse des Schaftes 1 einen spitzen Winkel ein, beispielsweise in der Größenordnung von 30°.

Durch die Neigung des oberen Teiles 2 des Schaftes und des daran anschließenden Ansatzes gegenüber dem unteren Teil 3 des Schaftes erhält die Endoprothese eine konvexe, außenliegende Seitenfläche 8 und eine innenliegende, konkave Seitenfläche 9, wobei diese Seitenflächen durch den Umfang des Schaftes 1 und durch die Seitenflächen des Ansatzes 4 gebildet werden.

Auf der konkaven Seitenfläche 9 ist dem oberen Teil 2 des Schaftes 1 folgend eine aus dieser Seitenfläche 9 vorstehende Rippe 10 angeformt, die sich vom oberen Ende des Schaftes 1 bis etwa zu dem Bereich erstreckt, in dem der obere Teil 2 in den unteren Teil 3 übergeht. Dabei nimmt die Höhe der Rippe 10 von oben nach unten kontinuierlich ab.

Die Rippe ist an der Seitenfläche 9 etwa in Höhe der Mittelachse des Schaftes 1 angeordnet, ihre maximale Höhe entspricht etwa dem halben Durchmesser des Schaftes 1.

In die obere Stirnseite 11 des Schaftes 1 ist eine Gewindesacklochbohrung 12 eingearbeitet, deren Längsachse parallel zur Längsachse des unteren Teiles 3 des Schaftes 1 verläuft. Neben der Gewindesacklochbohrung 12 befindet sich in der Stirnfläche 11 eine Vertiefung 13, die sich nicht über den gesamten Umfang der Gewindesacklochbohrung 12 erstreckt.

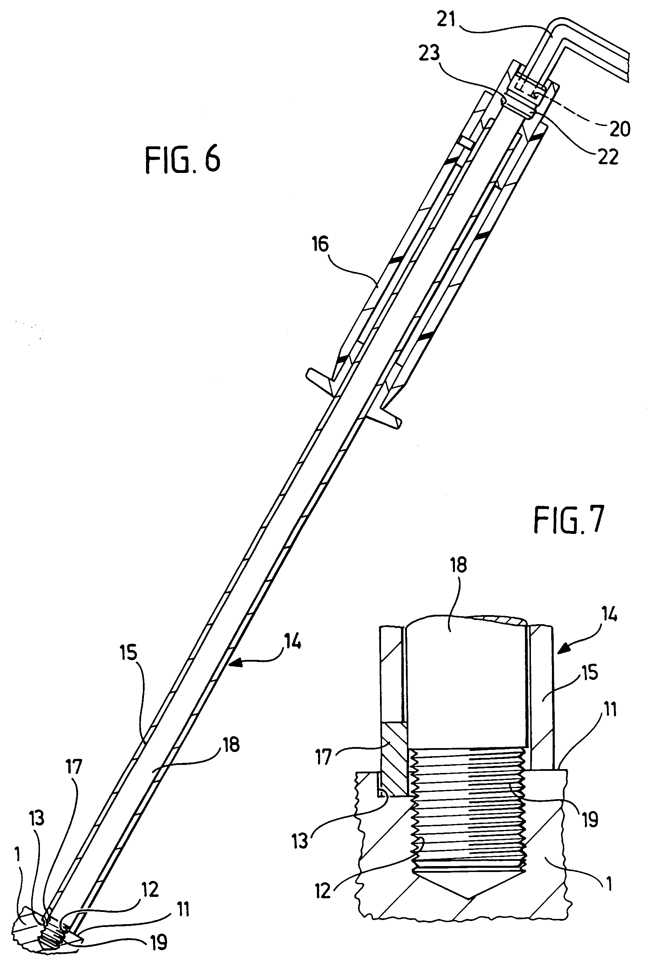

Die Gewindesacklochbohrung 12 und die Vertiefung 13 dienen der Aufnahme eines Einsetzwerkzeuges 14, das im folgenden anhand der Figuren 6 und 7 näher erläutert wird. Dieses Einsetzwerkzeug 14 umfaßt eine Hülse 15, die einseitig drehfest mit einem diese umgebenden Handgriff 16 verbunden ist. An der gegenüberliegenden Seite trägt die Hülse 15 einen in die Vertiefung 13 passenden Vorsprung 17.

Die Hülse 15 nimmt einen Stab 18 auf, der an seinem aus der Hülse 15 hervorstehenden Ende ein in die Gewindesacklochbohrung 12 passendes Gewinde 19 trägt. An der gegenüberliegenden Seite weist der Stab 18 eine Mehrkantinnenbohrung 20 auf, in die ein Mehrkantschlüssel 21 einsetzbar ist. Der Stab 18 liegt mit einer Erweiterung 22 am oberen Ende an einer Schulter 23 des Handgriffes 16 an.

Zur Festlegung des Einsetzwerkzeuges 14 an der Schaftendoprothese wird der Stab 18 mit dem Gewinde 19 in die Gewindesacklochbohrung 12 eingeschraubt, und zwar derart, daß dabei der Vorsprung 17 der Hülse 15 in die Vertiefung 13 eintaucht. Durch den eingeschraubten Stab 18 wird die Hülse 15 fest gegen die Endoprothese gepreßt, so daß der Stab 18 als Zuganker fungiert, der die Hülse wegen des Eingriffes des Vorsprunges 17 in die Vertiefung 13 drehfest mit der Endoprothese verbindet. Nach Abnahme des Mehrkantschlüssels 21, mit dem der Stab 18 eingedreht worden ist, ist dadurch mit der Schaftendoprothese ein Werkzeug starr verbunden, mit dem die Prothese eingesetzt oder auch wieder herausgezogen werden kann. Wichtig ist vor allen Dingen, daß durch Schläge auf den Handgriff keine Gefahr einer Verletzung des Gewindes in der Gewindesacklochbohrung 12 besteht, da die Schläge über die unmittelbar auf der Schaftendoprothese aufsitzende Hülse 15 auf diese übertragen werden. Ein weiterer Vorteil liegt darin, daß beim Lösen des Stabes 18 durch Festhalten des mit der Hülse drehfest verbundenen Handgriffes 16 eine Übertragung der Drehmomente auf die Schaftendoprothese vermieden wird. Es besteht also nicht die Gefahr, daß die Schaftendoprothese nach dem Einsetzen in den Knochen wieder gelockert wird.

## Patentansprüche

1. Schaftendoprothese für Röhrenknochen mit einem im Querschnitt kreisbogenförmigen Schaft (1), dessen Länge in einer ersten Ebene verläuft, mit einem an den oberen Teil (3) des Schaftes einseitig angeformten, sich in einer zweiten, senkrecht zur ersten verlaufenden Ebene erstreckenden und sich nach oben hin erweiternden Ansatz (4), der auf der dem Schaft abgewandten Seite bogenförmig begrenzt wird und dessen Dicke quer zur zweiten Ebene dem Durchmesser des Schaftes entspricht, und mit einem sich an die Oberseite des Ansatzes (4) anschließenden, in der zweiten Ebene liegenden, eine Gelenkkugel aufnehmenden, gegenüber der Längsachse des Schaftes geneigten Hals (6),
**dadurch gekennzeichnet,** daß der untere Teil (3) des Schaftes (1) gegenüber der zweiten Ebene um einen Winkel zwischen 5° und 15° geneigt ist und daß an den oberen Teil (3) des Schaftes (1) eine sich längs des Schaftes (1) und senkrecht zur zweiten Ebene über die konkave Außenfläche (9) erhebende Rippe (10) angeformt ist.

2. Endoprothese nach Anspruch 1, dadurch gekennzeichnet, daß der Winkel zwischen dem unteren Teil (3) des Schaftes (1) und der zweiten Ebene bei etwa 10° liegt.

3. Endoprothese nach Anspruch 1 oder 2, dadurch gekennzeichnet, daß der Schaft (1) im Übergangsbereich von seinem unteren geradlinigen Teil (3) in seinen oberen, in der zweiten Ebene liegenden Teil (2) kontinuierlich gebogen ist.

4. Endoprothese nach Anspruch 4, dadurch gekennzeichnet, daß auch der Teil des Ansatzes (4), der im Übergangsbereich angeordnet ist, in gleicher Weise wie der Schaft (1) kontinuierlich gebogen ist.

5. Endoprothese nach einem der voranstehenden Ansprüche, dadurch gekennzeichnet, daß der Übergang von dem geradlinigen unteren Teil (3) des Schaftes (1) in die dagegen geneigte zweite Ebene etwa in der Mitte der Endoprothese erfolgt.

6. Endoprothese nach einem der voranstehenden Ansprüche, dadurch gekennzeichnet, daß sich die Rippe (10) etwa bis zum Beginn des unteren, geradlinigen, gegenüber der zweiten Ebene geneigten Teil (3) des Schaftes (1) erstreckt.

7. Endoprothese nach Anspruch 6, dadurch gekennzeichnet, daß die Höhe der Rippe (10) gegenüber der Außenfläche (9) des Schaftes (1) von oben nach unten abnimmt.

8. Endoprothese nach einem der voranstehenden Ansprüche, dadurch gekennzeichnet, daß am oberen Ende des Schaftes (1) eine Verbindungsstelle (12, 13) für ein Einsetzwerkzeug (14) angeordnet ist.

## Claims

1. A shaft endoprosthesis for tubular bones, comprising a shaft (1) of circular cross-section, the length of which extends in a first plane, comprising an extension (4) which is formed on one side of the upper part (3) of the shaft, extends in a second plane perpendicular to the first plane and widens upwards, the extension (4) being curved on the side remote from the shaft and having a thickness, transversely to the second plane, which corresponds to the diameter of the shaft, and comprising a ball-receiving neck (6) which adjoins the upper end of the extension (4), lies in the second plane and is inclined relative to the longitudinal axis of the shaft,
characterised in that the lower part (3) of the shaft (1) is inclined at an angle of between 5° and 15° to the second plane, and in that a rib (10), projecting from the concave outer surface (9) and extending along the shaft (1) perpendicularly to the second plane, is formed on the upper part (3) of the shaft (1).

2. An endoprosthesis according to claim 1, characterised in that the angle between the lower part (3) of the shaft (1) and the second plane is approximately 10°.

3. An endoprosthesis according to claim 1 or 2, characterised in that the shaft (1) is continuously curved in the transition region from its lower straight part (3) to its upper part (2) lying in the second plane.

4. An endoprosthesis according to claim 4 [sic], characterised in that the part of the extension (4) arranged in the transition region is also continuously curved in the same manner as the shaft (1).

5. An endoprosthesis according to any one of the preceding claims, characterised in that the transition from the straight lower part (3) of the shaft (1) to the second plane inclined at an angle thereto occurs substantially in the middle of the endoprosthesis.

6. An endoprosthesis according to any one of the preceding claims, characterised in that the rib (10) extends substantially as far as the beginning of the lower straight part (3) of the shaft (1) inclined relative to the second plane.

7. An endoprosthesis according to claim 6, characterised in that the height of the rib (10) above the outer surface (9) of the shaft (1) decreases from top to bottom.

8. An endoprosthesis according to any one of the preceding claims, characterised in that a connection point (12, 13) for an insertion tool (14) is arranged at the upper end of the shaft (1).

## Revendications

1. Endoprothèse à tige pour os creux, comportant une tige (1) de section en forme d'arc de cercle, dont la longueur se trouve dans un premier plan, ainsi qu'un prolongement (4) qui est venu de forme, d'un côté, sur la partie supérieure (3) de la tige, s'étend dans un second plan, perpendiculaire au premier, s'élargit vers le haut, est délimité en forme d'arc du côté opposé à la tige et dont l'épaisseur, perpendiculairement au second plan correspond au diamètre de la tige, ainsi qu'un col (6) qui se raccorde à la face supérieure du prolongement (4), se trouve dans le second plan, reçoit une rotule mâle et est incliné par rapport à l'axe longitudinal de la tige,
caractérisée par le fait
que la partie inférieure (3) de la tige (1) est inclinée, par rapport au second plan, d'un angle valant entre 5° et 15° et qu'à la partie supérieure (3) de la tige (1) est venue de forme une nervure (10) qui dépasse au-dessus de la surface extérieure concave (9), le long de la tige (1) et perpendiculairement au second plan.

2. Endoprothèse selon la revendication 1, caractérisée par le fait que l'angle entre la partie inférieure (3) de la tige (1) et le second plan vaut environ 10°.

3. Endoprothèse selon la revendication 1 ou 2, caractérisée par le fait que, dans la zone de transition pour passer de sa partie inférieure rectiligne (3) dans sa partie supérieure (2), située dans le second plan, la tige (1) est cintrée de façon continue.

4. Endoprothèse selon la revendication 4, caractérisée par le fait qu'également, la partie du prolongement (4) qui est dis posée dans la zone de transition, est cintrée de façon continue, de la même façon que la tige (1).

5. Endoprothèse selon l'une des revendications précédentes, caractérisée par le fait que la transition pour passer de la partie inférieure rectiligne (3) de la tige (1) dans le second plan, incliné par rapport à elle, se fait à peu près au milieu de l'endoprothèse.

6. Endoprothèse selon l'une des revendications précédentes, caractérisée par le fait que la nervure (10) s'étend à peu près jusqu'au début de la partie inférieure, rectiligne, (3), inclinée par rapport au second plan, de la tige (1).

7. Endoprothèse selon la revendication 6, caractérisée par le fait que la hauteur de la nervure (10) par rapport à la surface extérieure (9) de la tige (1) décroît de haut en bas.

8. Endoprothèse selon l'une des revendications précédentes, caractérisée par le fait qu'à l'extrémité supérieure de la tige (1) est disposé un emplacement de liaison (12, 13) pour un outil de mise en place (14).
